# EUROPÄISCHE PATENTSCHRIFT

(11) **EP 0 600 290 B1**
(45) Veröffentlichungstag und Bekanntmachung des Hinweises auf die Patenterteilung: **14.10.1998**
(21) Anmeldenummer: 93118391.7
(22) Anmeldetag: 12.11.1993
(51) Int. Cl.: A61B 17/58

(54) **Vorrichtung zum Verbinden von Knochenteilen mittels einer Knochenplatte**
Apparatus to unite bone fragments by means of a bone plate
Dispositif de fixation de fragments osseux au moyen d'une plaque d'ostéosynthèse

(30) Priorität: 04.12.1992 DE 9216565 U
(43) Veröffentlichungstag der Anmeldung: 08.06.1994
(73) Patentinhaber: Waldemar Link GmbH & Co., 22339 Hamburg (DE)
(72) Erfinder: Eggers, Christoph, Dr., D-22609 Hamburg (DE); Keller, Arnold, D-23863 Kayhude (DE)
(74) Vertreter: Glawe, Delfs, Moll & Partner

(56) Entgegenhaltungen:
- EP-A- 0 016 270
- EP-A- 0 242 842
- EP-A- 0 392 927
- EP-A- 0 507 162
- DE-A- 2 718 515
- FR-A- 2 499 400
- US-A- 2 251 209

## Beschreibung

Es sind Knochenplattenanordnungen, insbesondere für die Wirbelsäulenchirurgie bekannt, die es gestatten, eine feste Winkelrelation zwischen einer Knochenschraube und einer Knochenplatte herzustellen, beispielsweise um einem Knochenfragment oder einem Wirbelkörper eine vorbestimmte Winkelstellung im Verhältnis zu benachbarten Fragmenten oder Wirbelkörpern zu geben (EP-B 0 201 024, EP-A 0 242 842, WO-A 90/12547, EP-A 0 507 162, FR-A 24 99 400, DE-A 27 18 515, EP-A 392 927, US-A 22 51 209, EP-A 16 270). Die bekannten Anordnungen sind kompliziert in der Anwendung und/oder unsicher hinsichtlich der Winkelfixierung und/oder platzraubend und/oder zu unflexibel, da die Winkelstellung vorgegeben ist und nicht vom Chirurgen an die Gegebenheiten angepaßt werden kann.

Diese Nachteile werden erfindungsgemäß durch die Merkmale des Anspruchs 1 sowie vorzugsweise der Unteransprüche beseitigt oder vermindert.

Die Erfindung wird im folgenden näher unter Bezugnahme auf die Zeichnung erläutert, die ein vorteilhaftes Ausführungsbeispiel veranschaulicht. Darin zeigen:

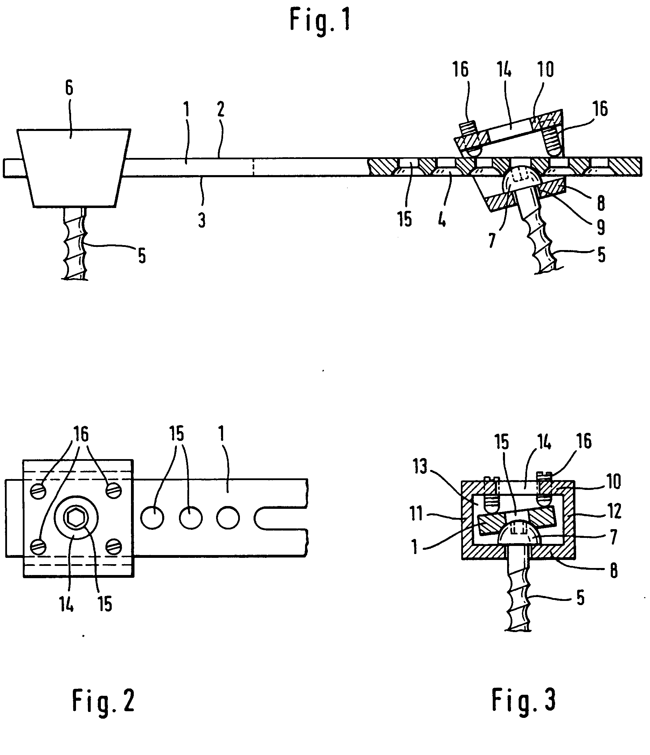

- Fig. 1: eine teilweise geschnittene Ansicht auf die Längsseite,
- Fig. 2: eine Draufsicht auf eine Hälfte der Vorrichtung und
- Fig. 3: einen Querschnitt.

Die schienenförmige Knochenplatte 1 hat eine ebene Oberseite 2. Die Unterseite 3 enthält zumindest an jedem Ende eine Reihe von mittigen, sphärischen Vertiefungen 4 sowie gewünschtenfalls im zentralen Bereich ein Langloch, das es gestattet, zwischen zwei mit den Enden der Knochenplatte zu verbindenden Knochenfragmenten oder Wirbelkörpern ein weiteres Knochenfragment oder einen Wirbelkörper oder mehrere Fragmente zu fixieren. Die Knochenplatte kann gewünschtenfalls in bekannter Weise zur Anpassung an einen gewünschten Knochenverlauf gebogen werden. Im allgemeinen ist dies jedoch dank der erfindungsgemäß ermöglichten Winkelverstellbarkeit der Knochenschrauben nicht erforderlich. Die Knochenplatte kann auch gewünschtenfalls von vornherein eine irgendwie geartete Biegung aufweisen.

Zur Verbindung der Knochenplatte mit einem Knochenfragment oder Wirbelkörper dienen Knochenschrauben 5, die mittels eines Kupplungsteils 6 mit der Knochenplatte verbunden werden. Dabei ist vorgesehen, daß die Winkelrelation der Knochenschraube 5 gegenüber dem Kupplungsteil 6 unvariabel vorbestimmt ist. Dies kann beispielsweise dadurch geschehen, daß die Knochenschraube und der Kupplungsteil einstückig miteinander verbunden sind oder daß ihre zusammenwirkenden Oberflächen einander in der Form so angepaßt sind, daß sich die vorbestimmte Winkelrelation ergibt. Letzteres ist in dem dargestellten Beispiel der Fall, wo die ebene Unterseite des Schraubenkopfs 7 auf die ebene Oberseite der Bodenplatte 8 des Kupplungsteils 6 gepreßt wird. Auch können der Schraubenschaft und die ihn aufnehmende Bohrung 9 des Kupplungsteils so ausgebildet sein, daß sie die gewünschte Winkelrelation sichern. Diese Winkelrelation ist im vorliegenden Fall dadurch gekennzeichnet, daß die Achse der Knochenschraube 5 lotrecht zu der Bodenplatte 8 des Kupplungsteils 6 steht.

Der Kupplungsteil 6 weist eine zu der Bodenplatte 8 parallele Deckplatte 10 auf, die mit der Bodenplatte durch zwei Seitenwände 11, 12 starr verbunden ist. Dadurch bildet sich zwischen der Deckplatte, der Bodenplatte und den Seitenwänden ein an beiden Enden offener Kanal 13, der die Knochenplatte 1 aufnimmt. Die Deckplatte 10 enthält eine Bohrung 14, durch die der Schraubenkopf 7 für ein Schraubendrehwerkzeug zugänglich ist. Im dargestellten Beispiel ist vorgesehen, daß auch die Knochenplatte 1 jeweils konzentrisch zu den unterseitigen Ausnehmungen 4 Bohrungen 15 enthält, durch die hindurch ein Schraubendrehwerkzeug Zugang zum Schraubenkopf 7 hat, damit die Knochenschraube 5 auch dann gedreht werden kann, wenn sich die Knochenplatte 1 in dem Kupplungsteil befindet. Unbedingt erforderlich ist dies jedoch nicht, da in vielen Fällen die Knochenschraube 5 mit dem zugehörigen Kupplungsteil 6 in das zugeordnete Knochenfragment eingeschraubt werden kann, bevor die Knochenplatte 1 in den Kupplungsteil 6 eingefügt wird.

Für die Fixierung der Knochenplatte 1 in dem Kupplungsteil 6 sind in der Deckplatte 10 des Kupplungsteils 6 vier Madenschrauben 16 vorgesehen, deren Achsen etwa lotrecht zur Ebene der Deckplatte 10 und daher auch ungefähr lotrecht zur Erstreckung der Knochenplatte 1 stehen. Sie befinden sich nach verschiedenen Seiten hin in Abstand von dem Schraubenkopf 7 und in den Eckpunkten eines gedachten Rechtecks, dessen Seiten parallel bzw. quer zur Erstreckungsrichtung der Knochenplatte verlaufen. Außerdem befinden sie sich innerhalb der seitlichen Grenzen der Knochenplatte 1, so daß sie, wenn sie in den Kupplungsteil eingeschraubt werden, schließlich die Oberseite 2 der Knochenplatte treffen, und zwar bei jeder praktisch vorkommenden Winkeleinstellung der Knochenplatte zum Kupplungsteil. Geometrisch würden drei Schrauben ausreichen, aber diese Zahl ist schwer mit den konstruktiven Voraussetzungen in Einklang zu bringen.

Diese Winkelstellung ist dadurch variabel, daß die Knochenplatte über ein Schwenklager, das eine Schwenkbewegung um die Querachse und/oder um die Längsachse der Knochenplatte ermöglicht, von der Bodenplatte 8 gestützt ist. Dieses Schwenklager wird im dargestellten Beispiel dadurch gebildet, daß jeweils eine ausgewählte unterseitige Vertiefung 4 der Knochenplatte mit der Oberfläche des Schraubenkopfs 7 zusammenwirkt, die im wesentlichen übereinstimmend sphärisch mit den Ausnehmungen 4 ausgebildet ist. Die gewünschte Winkeleinstellung der Knochenplatte zu dem Kupplungsteil wird dann mittels der Schrauben 16 eingestellt, indem diese mehr oder weniger weit in den Kupplungsteil eingeschraubt werden.

Diese Anordnung gibt eine außerordentlich feste Verbindung zwischen der Knochenplatte und dem Kupplungsteils, weil einerseits die Knochenplatte in Längs- und Querrichtung über die mit dem Schraubenkopf 7 zusammenwirkende Ausnehmung 4 gegenüber dem Kupplungsteils verriegelt ist und andererseits die Schrauben 16 die Winkelstellung eindeutig und unveränderbar bestimmen. Da die Knochenplatte sich gegenüber der Kraft der Schrauben 16 an dem Schraubenkopf 7 abstützt, wird dieser gegen die Bodenplatte 8 des Kupplungsteils 6 gepreßt und daher gleichfalls festgelegt.

Die Knochenplatte kann auch in anderer Weise als mit den erfindungsgemäßen Kupplungsteilen verwendet werden. Beispielsweise kann sie mittels der Bohrungen 15 unmittelbar an einem Ende mit einem Knochenfragment verschraubt werden, während sie am anderen Ende über ein Kupplungsteil 6 mit dem dortigen Knochenfragment zusammenwirkt. Es ist auch nicht erforderlich, daß die Knochenplatte unterseitige Ausnehmungen 4 aufweist, da auch die untere Kante der Bohrungen 15 zur Bildung eines Schwenklagers mit dem Schraubenkopf 7 in der Lage ist. Die Unterseite der Knochenplatte kann auch gänzlich glatt (ohne Ausnehmungen 4 und ohne Bohrungen 15) ausgebildet sein, da sich auch dann in jedem beliebigen Punkt der Unterfläche ein Schwenklager mit dem Schraubenkopf 7 ergibt. Auch ist nicht erforderlich, daß der kupplungsseitige Teil des Schwenklagers von dem Schraubenkopf 7 gebildet wird; vielmehr könnte dafür irgendeine andere geeignete Erhebung innerhalb des Kupplungsteils, beispielsweise eine konische Spitze, vorgesehen sein. Die Knochenschraube (oder auch eine Gruppe von Knochenschrauben) könnte dann in anderer Weise mit der Bodenplatte des Kupplungsteils verbunden sein. Schließlich braucht die Deckplatte 10 des Kupplungsteils nicht starr mit der Bodenplatte 8 verbunden zu sein; vielmehr kann es sich dabei um eine gesonderte Platte handeln, die mittels mehrerer außerhalb der seitlichen Grenzen der Knochenplatte 1 liegender Schrauben mit der Bodenplatte verbunden ist, die unterschiedlich weit in die Bodenplatte eingeschraubt werden, um der großflächig eben von der Deckplatte 10 beaufschlagten Knochenplatte 1 die gewünschte Winkelstellung gegenüber der Bodenplatte 8 zu verleihen.

## Patentansprüche

1. Vorrichtung zum Verbinden von Knochenteilen, die eine Knochenplatte (1), eine Knochenschraube (5) und einen Kupplungsteil (6) zum winkelfesten Verbinden der Knochenschraube (5) mit der Knochenplatte (1) umfaßt, dadurch gekennzeichnet, daß der Kupplungsteil (6) winkelfest mit der Knochenschraube (5) verbindbar oder verbunden ist und einerseits mit der Plattenunterseite (3) ein Schwenklager bildet und andererseits eine Deckplatte (10) aufweist, die mindestens drei in seitlicher Versetzung gegenüber dem Schwenklager angeordnete, auf die Plattenoberseite (2) wirkende Fixierschrauben (16) enthält und starr mit der das Schwenklager tragenden Bodenplatte (8) verbunden ist, wobei der Kupplungsteil (6) die Knochenplatte (1) quer umfaßt und die Knochenplatte zwischen der Deckplatte (10) und der Bodenplatte (8) angeordnet ist.

2. Vorrichtung nach Anspruch 1, dadurch gekennzeichnet, daß der eine Teil des Schwenklagers von dem Kopf (7) der Knochenschraube (5) gebildet ist, für deren Schaft in der Bodenplatte (8) eine Aufnahmebohrung (9) vorgesehen ist.

3. Vorrichtung nach Anspruch 1 oder 2, dadurch gekennzeichnet, daß auf der Unterseite (3) der Knochenplatte (1) mindestens eine Vertiefung (4) zur Bildung eines Schwenklagerteils vorgesehen ist.

4. Vorrichtung nach Anspruch 3, dadurch gekennzeichnet, daß zumindest eines der beiden an der Knochenplatte (1) und dem Kupplungsteil (6) zusammenwirkenden Schwenklagerteile sphärisch ausgebildet ist.

5. Vorrichtung nach Anspruch 3 oder 4, dadurch gekennzeichnet, daß die Knochenplatte (1) am Ort der Vertiefung (4) eine Werkzeugöffnung (15) aufweist.

6. Vorrichtung nach einem der Ansprüche 1 bis 5, dadurch gekennzeichnet, daß die Deckplatte (10) des Kupplungsteils (6) durch zwei die Knochenplatte (1) einfassende Wände (11,12) mit der Bodenplatte (8) verbunden ist.

7. Vorrichtung nach einem der Ansprüche 1 bis 6, dadurch gekennzeichnet, daß vier rixierschrauben (16) vorgesehen sind, deren Verbindungslinien parallel bzw. quer zur Richtung der Knochenplatte (1) verlaufen.

## Claims

1. A device for connecting bone parts, which comprises a bone plate (1), a bone screw (5) and a coupling member (6) for the connection of the bone screw (5), at a fixed angle, to the bone plate (1), characterised in that the coupling member (6) is or can be connected at a fixed angle to the bone screw (5) and, on the one hand, forms a pivot bearing with the plate underside (3) and, on the other hand, has a cover plate (10) which contains at least three fixing screws (16) which are arranged laterally offset relative to the pivot bearing and which act on the upper side (2) of the plate, said cover plate being rigidly connected with the base plate (8) carrying the pivot bearing, wherein the coupling member (6) transversely embraces the bone plate (1) and the bone plate is disposed between the cover plate (10) and the base plate (8).

2. A device according to Claim 1, characterised in that one part of the pivot bearing is formed by the head (7) of the bone screw (5), for the shank of which an accommodating bore (9) is provided in the base plate (8).

3. A device according to Claim 1 or 2, characterised in that on the underside (3) of the bone plate (1) at least one recess (4) is provided to form a pivot bearing member.

4. A device according to Claim 3, characterised in that at least one of the two pivot bearing parts cooperating on the bone plate (1) and the coupling member (6) is spherically shaped.

5. A device according to Claim 3 or 4, characterised in that at the location of the recess (4) the bone plate (1) has an implement opening (15).

6. A device according to any one of Claims 1 to 5, characterised in that the cover plate (10) of the coupling member (6) is connected to the base plate (8) by two walls (11,12) enclosing the bone plate (1).

7. A device according to any one of claims 1 to 6, characterised in that four fixing screws (16) are provided, the connecting lines of which extend parallel or transversely to the direction of the bone plate (1).

## Revendications

1. Dispositif destiné à assembler des fragments osseux, qui comporte une plaque d'ostéosynthèse (1), une vis à os (5) et un élément d'accouplement (6) pour l'assemblage sous un angle fixe, de la vis à os (5) avec la plaque d'ostéosynthèse (1), caractérisé en ce que l'élément d'accouplement (6) peut être assemblé ou est assemblé sous un angle fixe avec la vis à os (5) et d'une part, forme un palier d'orientation avec la face inférieure (3) de la plaque et comporte, d'autre part, une plaque de couverture (10) qui contient au moins trois vis de fixation (16) décalées latéralement par rapport au palier d'orientation, agissant sur la face supérieure (2) de la plaque d'ostéosynthèse, et qui est reliée rigidement à la plaque de fond (8) portant le palier d'orientation, l'élément d'accouplement (6) entourant transversalement la plaque d'ostéosynthèse (1) et la plaque d'ostéosynthèse étant disposée entre la plaque de couverture (10) et la plaque de fond (8).

2. Dispositif selon la revendication 1, caractérisé en ce qu'une partie du palier d'orientation est formée par la tête (7) de la vis à os (5), pour la tige de laquelle un trou de passage (9) est prévu dans la plaque de fond (8).

3. Dispositif selon la revendication 1 ou 2, caractérisé en ce que sur la face inférieure (3) de la plaque d'ostéosynthèse (1) est prévu au moins un évidement (4) destiné à former une partie du palier d'orientation.

4. Dispositif selon la revendication 3, caractérisé en ce qu'au moins l'une des deux parties coopérantes du palier d'orientation formé sur la plaque d'ostéosynthèse (1) et l'élément d'accouplement (6), est sphérique.

5. Dispositif selon la revendication 3 ou 4, caractérisé en ce que la plaque d'ostéosynthèse (1) présente une ouverture (15) pour un outil à l'emplacement de l'évidement (4).

6. Dispositif selon l'une des revendications 1 à 5, caractérisé en ce que la plaque de couverture (10) de l'élément d'accouplement (6) est reliée à la plaque de fond (8) par deux parois (11, 12) enserrant la plaque d'ostéosynthèse (1).

7. Dispositif selon l'une des revendications 1 à 6, caractérisé en ce que sont prévues quatre vis de fixation (16) dont les lignes de liaison s'étendent parallèlement ou transversalement à la direction de la plaque d'ostéosynthèse (1).
